# EUROPEAN PATENT APPLICATION

(11) **EP 4 785 976 A2**
(43) Date of publication of application: **05.08.2026**
(21) Application number: 26179763.3
(22) Date of filing: 13.05.2022
(51) Int. Cl.: A61M 5/158

(54) **CATHETER ASSEMBLY HAVING A SIDE PORT PATHWAY**

(30) Priority: 14.05.2021 US 202163188834 P
(62) Divisional of application: 22808386.1
(71) Applicant: Becton, Dickinson and Company, Franklin Lakes, NJ 07417 (US)
(72) Inventor: LACKEY, John, West Valley City, Utah 84119 (US); BURKHOLZ, Jonathan, Karl, Salt Lake City, Utah 84108 (US); MA, Yiping, Layton, Utah 84040 (US); LACKEY, BreAnna, West Valley City, Utah 84119 (US); SONDEREGGER, Ralph, L., Farmington, Utah 84025 (US); MITCHELL, Nathan, Murray, Utah 84123 (US); SCHERICH, Megan, Salt Lake City, Utah 84115 (US); BLANCHARD, Curtis, H., Riverton, Utah 84096 (US); BOUD, Adam, J., Bluffdale, Utah 84065 (US); ELEY, Taylor, Alpharetta, Georgia 30005 (US); LAUER, Shaun, Portland, Oregon 97203 (US)
(74) Representative: dompatent

(57) **Abstract**

A catheter adapter may include a distal end, a proximal end, an inner surface extending through the distal end and the proximal end and forming a lumen, and a side port forming a side port pathway through a sidewall of the catheter adapter and in fluid communication with the lumen. The catheter assembly may include a catheter extending distally from the distal end of the catheter adapter. The catheter assembly may include a septum disposed within the lumen proximal to the side port pathway. The catheter assembly may include one or more features to facilitate flushing of the catheter assembly: a diameter of the side port may increase in a distal direction; a portion of the inner surface proximal to the catheter and distal to the septum may include multiple grooves; the side port pathway may include a non-cylindrical portion; an insert; a rotation element; and a pivot element.

## Description

### CROSS-REFERENCE TO RELATED APPLICATION

The present application aspects priority to United States Provisional Application Serial No. 63/188,834, entitled "Catheter Assembly Having a Side Port Pathway and Related Methods", filed May 14, 2021, the entire disclosure of which is hereby incorporated by reference in its entirety.

### BACKGROUND OF THE INVENTION

A catheter is commonly used to infuse fluids into vasculature of a patient. For example, the catheter may be used for infusing normal saline solution, various medicaments, or total parenteral nutrition. The catheter may also be used for withdrawing blood from the patient.

The catheter may include an over-the-needle peripheral intravenous ("IV") catheter. In this case, the catheter may be mounted over an introducer needle having a sharp distal tip. The catheter and the introducer needle may be assembled so that the distal tip of the introducer needle extends beyond the distal tip of the catheter with the bevel of the needle facing up away from skin of the patient. The catheter and introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of a catheter assembly that includes the catheter. After placement of the needle has been confirmed, the clinician may remove the needle, leaving the catheter in place for future blood withdrawal or fluid infusion.

Many catheter assemblies have a septum proximal to an extension tube inlet, creating a region distal to the septum that is difficult to flush free of fluids (such as blood or infusates). Stagnant fluid within the region distal to the septum may lead to accumulation of bacteria within the catheter assembly, which may result in infection or removal of the catheter from the patient.

The subject matter aspected herein is not limited to embodiments that solve any disadvantages or that operate only in environments such as those described above. Rather, this background is only provided to illustrate one example technology area where some implementations described herein may be practiced.

### SUMMARY OF THE INVENTION

The present disclosure relates generally to vascular access devices, systems, and methods. More particularly, the present disclosure relates to a catheter assembly having a side port pathway, as well as related devices and methods. In some embodiments, the catheter assembly may include a catheter adapter, which may include a distal end, a proximal end, and an inner surface extending through the distal end and the proximal end. In some embodiments, the inner surface of the catheter adapter may form a lumen. In some embodiments, the catheter adapter may include a side port forming a side port pathway through a sidewall of the catheter adapter and in fluid communication with the lumen.

In some embodiments, the catheter assembly may facilitate flushing of the catheter assembly to avoid stagnant fluid within the catheter assembly. In some embodiments, the side port pathway of the catheter assembly may include one or more features to direct fluid flow and induce turbulent fluid flow within the catheter assembly, which may facilitate flushing. In some embodiments, the features of the side port pathway may facilitate flushing of a region within the catheter assembly that may otherwise be difficult to flush free of fluids (such as blood or infusates). In some embodiments, the region may be disposed distal to the septum. Stagnant fluid within the region distal to the septum may lead to accumulation of bacteria within the catheter assembly, which may result in infection or removal of the catheter from the patient.

In some embodiments, the catheter assembly may include an extension tube integrated within the side port. In some embodiments, a diameter of the side port pathway may increase in a distal direction. In some embodiments, the catheter assembly may include a catheter extending distally from the distal end of the catheter adapter. In some embodiments, the catheter may include a peripheral intravenous catheter, a peripherally-inserted central catheter, or a midline catheter. In some embodiments, the catheter assembly may include a septum, which may be disposed within the lumen proximal to the side port pathway.

In some embodiments, the side port pathway may include a first side and a second side opposite the first side. In some embodiments, the first side may be straight. In some embodiments, a proximal end of the second side may be straight, and a distal end of the second side may be angled proximally with respect to the proximal end of the second side. In some embodiments, the first side may be distal to the second side. In some embodiments, the distal end of the second side may contact the septum.

In some embodiments, the septum may include an elastomeric body surrounded by a canister. In some embodiments, the septum and catheter assembly may not include the canister. In some embodiments, the septum may include a one-piece septum, a two-piece septum, or another suitable septum.

In some embodiments, the side port pathway may include a non-cylindrical portion. In some embodiments, the non-cylindrical portion may include multiple grooves or ribs generally parallel to a central axis of the side port pathway. In some embodiments, the grooves or ribs may be evenly spaced around an inner circumference of the catheter adapter. In some embodiments, the catheter assembly may include the extension tube integrated with the side port. In some embodiments, each of the grooves or ribs may include a proximal end proximate the extension tube. In some embodiments, each of the grooves or ribs may include a distal end proximate the lumen.

In some embodiments, the non-cylindrical portion may include a helical groove. In some embodiments, a distal end of the helical groove may be proximate the septum. In some embodiments, the catheter assembly may include the extension tube integrated with the side port, and a proximal end of the helical groove is proximate the extension tube.

In some embodiments, the non-cylindrical portion may include multiple ridges, which may be generally perpendicular to a central axis of the side port pathway. In some embodiments, the ridges may be evenly spaced apart. In some embodiments, the ridges may be annular.

In some embodiments, the non-cylindrical portion may include a narrowed diameter portion. In some embodiments, the narrowed diameter portion may include a first diameter. In some embodiments, the side port pathway may include a second diameter distal to the first diameter and a third diameter proximal to the first diameter. In some embodiments, the second diameter and the third diameter may be greater than the first diameter. In some embodiments, the non-cylindrical portion may be disposed within the extension tube, which may be disposed within the side port pathway. Thus, in some embodiments, the non-cylindrical portion may be disposed within the side port pathway in this manner. In some embodiments, the extension tube may extend into or through the side port pathway.

In some embodiments, the catheter assembly may include an insert disposed at least partially within the side port pathway and configured to divert fluid flow. In some embodiments, the insert may extend into the lumen from the side port pathway and may include one or more holes. In some embodiments, a distal end of the insert may include a corkscrew shape. In some embodiments, the insert may include a groove extending along a longitudinal axis of the insert. In some embodiments, the insert may be formed by a distal end of the extension tube, which may extend into or through the side port pathway and which may divert fluid flow.

In some embodiments, the insert may include a protrusion on the inner surface of the catheter adapter. In some embodiments, the protrusion may divide the side port pathway into a first fluid pathway extending in a distal direction and a second fluid pathway extending in a proximal direction.

In some embodiments, the catheter assembly may include a fluid diverter disposed between the septum and the catheter and aligned with the side port pathway. In some embodiments, the fluid diverter may include a rotation element, which may include a straight pathway extending therethrough. In some embodiments, the rotation element may be a cylinder or a sphere. In some embodiments, the rotation element may be configured to rotate in response to infusion of fluid from the side port pathway to the lumen. In some embodiments, the fluid diverter may include a pivot element coupled to the inner surface and configured to pivot in response to infusion of fluid from the side port pathway to the lumen.

It is to be understood that both the foregoing general description and the following detailed description are examples and explanatory and are not restrictive of the invention, as aspected. It should be understood that the various embodiments are not limited to the arrangements and instrumentality illustrated in the drawings. It should also be understood that the embodiments may be combined, or that other embodiments may be utilized and that structural changes, unless so aspected, may be made without departing from the scope of the various embodiments of the present invention. The following detailed description is, therefore, not to be taken in a limiting sense.

### BRIEF DESCRIPTION OF THE DRAWINGS

Example embodiments will be described and explained with additional specificity and detail through the use of the accompanying drawings in which:
Figure 1 is a cross-sectional view of a prior art catheter assembly;
Figure 2 is a cross-sectional view of an example catheter assembly, illustrating a diameter of an example side port pathway increasing in a distal direction, according to some embodiments;
Figure 3 is a cross-sectional view of the catheter assembly, illustrating an example diverging feature to induce rotational flow, according to some embodiments;
Figure 4A is a cross-sectional view of the catheter assembly, illustrating example grooves, according to some embodiments;
Figure 4B is a transverse cross-sectional view of the catheter assembly through the grooves, according to some embodiments;
Figure 5 is a cross-sectional view of the catheter assembly, illustrating an example helical groove, according to some embodiments;
Figure 6 is a cross-sectional view of the catheter assembly, illustrating example ridges, according to some embodiments; and
Figure 7 is a cross-sectional view of the catheter assembly, illustrating an example narrowed diameter portion, according to some embodiments;
Figure 8A is a cross-sectional view of the catheter assembly, illustrating an example insert, according to some embodiments;
Figure 8B is a partial cutaway view of the catheter assembly, illustrating the insert of Figure 8A, according to some embodiments;
Figure 9A is a partial cutaway view of the catheter assembly, illustrating another example insert, according to some embodiments;
Figure 9B is a cross-sectional view of the catheter assembly through an example side port pathway proximate the insert of Figure 9A, according to some embodiments;
Figure 10A is a cross-sectional view of the catheter assembly, illustrating another example insert, according to some embodiments;
Figure 10B is a partial cutaway view of the catheter assembly, illustrating the insert of Figure 10A, according to some embodiments;
Figure 11A is a cross-sectional view of the catheter assembly, illustrating another example insert, according to some embodiments;
Figure 11B is a partial cutaway view of the catheter assembly, illustrating the insert of Figure 11A, according to some embodiments;
Figure 12A is a partial view of the catheter assembly, illustrating another example insert, according to some embodiments;
Figure 12B is a cross-sectional view of the catheter assembly through an example side port pathway and the insert of Figure 12A, according to some embodiments;
Figure 13 is a cross-sectional view of the catheter assembly, illustrating another example insert, according to some embodiments;
Figure 14A is a cross-sectional view of the catheter assembly, illustrating an example rotation element, according to some embodiments;
Figure 14B is an upper perspective view of the rotation element of Figure 14A, according to some embodiments;
Figure 14C is a cross-sectional view of the catheter assembly, illustrating another example rotation element, according to some embodiments;
Figure 14D is a partial cutaway view of the catheter assembly, illustrating the other rotation element, according to some embodiments;
Figure 15A is a cross-sectional view of the catheter assembly, illustrating an example pivot element, according to some embodiments; and
Figure 15B is a cross-sectional view of the catheter assembly, illustrating another example pivot element, according to some embodiments.

### DETAILED DESCRIPTION

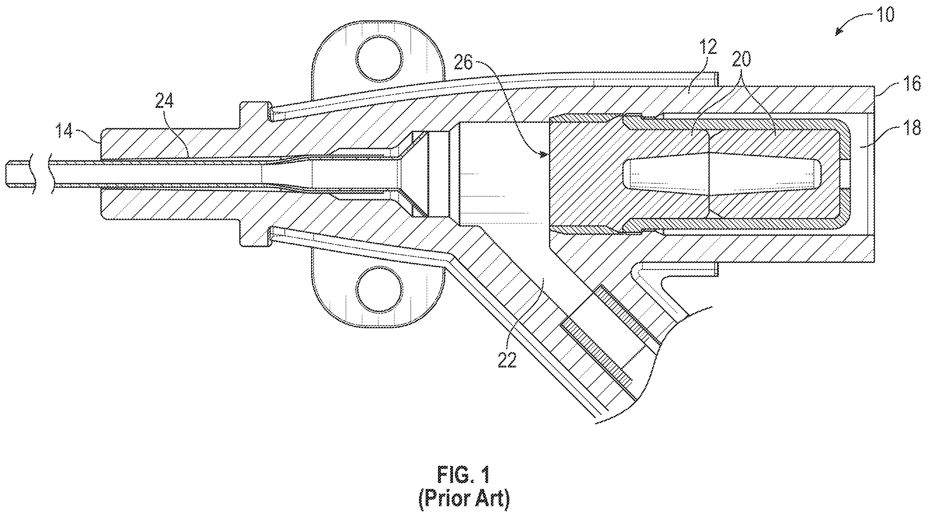

The present disclosure relates generally to vascular access devices, systems, and methods. More particularly, the present disclosure relates to a catheter assembly, as well as related devices and methods. Referring now to Figure 1, a prior art catheter assembly 10 is illustrated. The prior art catheter assembly 10 includes a catheter adapter 12 with a distal end 14, a proximal end 16, and a lumen 18 extending through the distal end 14 and the proximal end 16. The prior art catheter assembly 10 includes a septum 20 spaced apart and proximal to a side port pathway 22. A catheter 24 extends distally from the distal end 14. The prior art catheter assembly includes a region 26 in which fluid may become stagnant despite flushing through the side port pathway 22. The region 26 includes a distal face of the septum 20 and an area within the lumen 18 adjacent thereto. Stagnant fluid within the region 26 may lead to an accumulation of bacteria within the prior art catheter assembly 10, which may result in infection or removal of the catheter 24 from the patient.

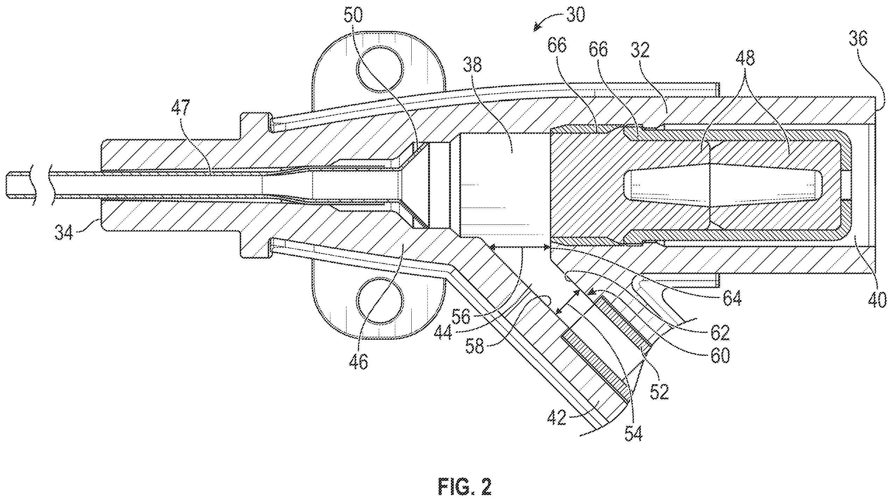

Referring now to Figure 2, a catheter assembly 30 is illustrated, according to some embodiments. In some embodiments, the catheter assembly 30 may include a catheter adapter 32, which may include a distal end 34, a proximal end 36, and an inner surface 38 extending through the distal end 34 and the proximal end 36. In some embodiments, the inner surface of the catheter adapter 32 may form a lumen 40. In some embodiments, the catheter adapter 32 may include a side port 42 forming a side port pathway 44 through a sidewall 46 of the catheter adapter 32 and in fluid communication with the lumen 40. In some embodiments, the sidewall 46 may include the inner surface 38. In some embodiments, the side port pathway 44 may extend from the lumen 40 to an outer opening of the side port 42.

In some embodiments, the catheter assembly 30 may include a catheter 47 extending distally from the distal end 34 of the catheter adapter 32. In some embodiments, the catheter 47 may include a peripheral intravenous catheter, a peripherally-inserted central catheter, or a midline catheter. In some embodiments, the catheter assembly 30 may include a wedge 50, which may secure the catheter 47 within the distal end 34 of the catheter adapter 32. In some embodiments, the catheter assembly 30 may include a septum 48, which may be disposed within the lumen 40 proximal to the side port pathway 44.

In some embodiments, the catheter assembly 30 may facilitate flushing of the catheter assembly 30 to avoid stagnant fluid within the catheter assembly 30. In some embodiments, the side port pathway 44 of the catheter assembly 30 may include one or more features to direct fluid flow and induce turbulent fluid flow within the catheter assembly 30, which may facilitate flushing. In some embodiments, the features of the side port pathway 44 may facilitate flushing of a region within the catheter assembly 30 that may otherwise be difficult to flush free of fluids (such as blood or infusates). In some embodiments, the region may be disposed distal and/or proximate to the septum 48. Stagnant fluid within the region distal to the septum 48 may lead to accumulation of bacteria within the catheter assembly 30, which may result in infection or removal of the catheter from the patient. However, the features of the side port pathway 44 may facilitate fluid turbulence within the region, decreasing a likelihood of accumulation of bacteria. In some embodiments, the catheter assembly 30 may reduce build-up of blood on the inner surface 38 and/or reduce interactions of incompatible fluids.

In some embodiments, the catheter assembly may include an extension tube 52 integrated within the side port 42. In some embodiments, the side port pathway 44 may be used for fluid infusion and/or blood collection. In some embodiments, a proximal end of the extension tube 52 may be coupled to a blood collection device.

In some embodiments, a diameter of the side port pathway 44 may increase in a distal direction. For example, a proximal portion of the side port pathway 44 may include a first diameter 54, and a distal portion of the side port pathway 44 may include a second diameter 56, which may be greater than the first diameter 54.

In some embodiments, the side port pathway 44 may include a first side 58 and a second side 60 opposite the first side 58. In some embodiments, the first side 58 may be straight or a have a linear cross-section, as illustrated, for example, in Figure 2. In some embodiments, a proximal end 62 of the second side 60 may be straight, and a distal end 64 of the second side 60 may be angled proximally with respect to the proximal end 62 of the second side 60. Thus, in some embodiments, the second side 60 may be diverging, which may facilitate flushing of the region in front or distal to the septum 48 that may otherwise be difficult to flush free of fluids. In some embodiments, the first side 58 may be distal to the second side 60. In some embodiments, the distal end 64 of the second side 60 may be angled between 0° and 90° with respect to the proximal end 62 of the second side 60. In some embodiments, the distal end 64 of the second side 60 may be angled between 20° and 60°, inclusive, with respect to the proximal end 62 of the second side 60. In some embodiments, the distal end 64 of the second side 60 may contact the septum 48. In some embodiments, a distal end of the first side 58 may be diverging and angled distally with respect to a proximal end of the first side 58, which may increase flushability of the region.

In some embodiments, the septum 48 may include an elastomeric body surrounded by a canister 66. In some embodiments, the septum 48 and catheter assembly 30 may not include the canister 66. In some embodiments, the septum 48 may include a one-piece septum, a two-piece septum, or another suitable septum.

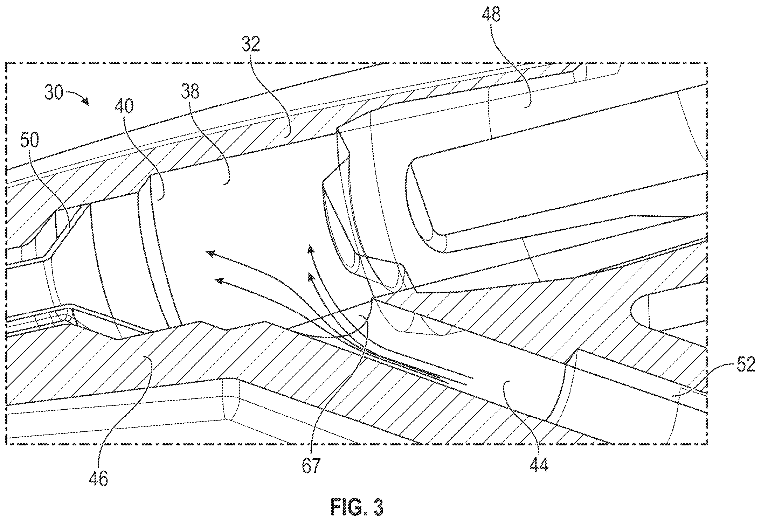

Referring now to Figure 3, in some embodiments, a wall of the side port pathway may include a diverging feature, which may induce rotational flow of fluid flowing into the lumen 40 from the side port pathway 44 and facilitate flushing of the region in front or distal to the septum 48. In some embodiments, the diverging feature may include a protrusion disposed at an intersection of the side port pathway 44 and the lumen 40. In some embodiments, the protrusion may include a stepped surface or wall 67 that may extend across a width of the side port pathway 44.

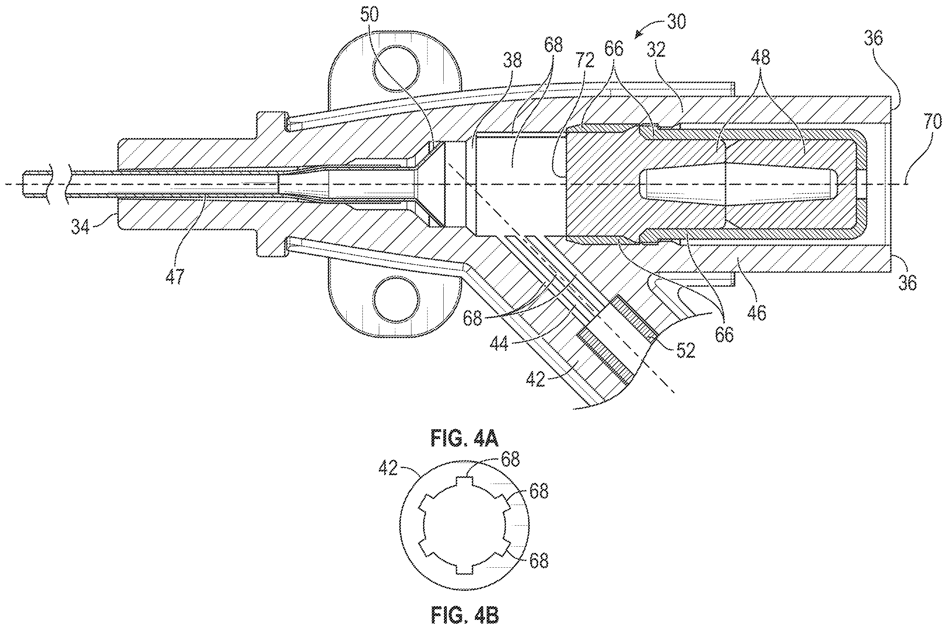

Referring now to Figures 4A-4B, in some embodiments, the side port pathway 44 may include a non-cylindrical portion. In some embodiments, the non-cylindrical portion may include multiple grooves 68. In some embodiments, the grooves 68 may be generally parallel to a central axis of the side port pathway 44, which may intersect a longitudinal axis 70 of the catheter assembly 30. In some embodiments, the grooves 68 may be evenly spaced around an inner circumference of the side port 42. In some embodiments, each of the grooves 68 may include a distal end proximate or adjacent the lumen 40 and/or a distal end of the septum 48. In some embodiments, each of the grooves 68 may include a proximal end proximate or adjacent the extension tube 52. In some embodiments, the grooves 68 may be replaced with ribs. In some embodiments, the grooves 68 or the ribs may direct fluid flow to facilitate flushing of the region distal to the septum 48. In some embodiments, the grooves 68 may be molded using a finned core pin.

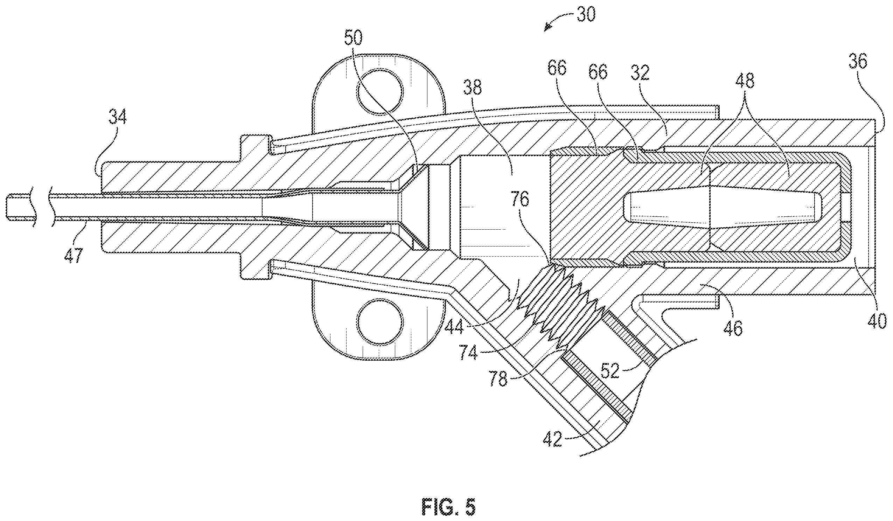

Referring now to Figure 5, in some embodiments, the side port pathway 44 may include a non-cylindrical portion. In some embodiments, the non-cylindrical portion may include a helical groove 74. In some embodiments, the helical groove 74 may wind helically around the inner surface 38, which may be generally cylindrical. In some embodiments, a distal end 76 of the helical groove 74 may be proximate the septum 48. In some embodiments, the catheter assembly 30 may include the extension tube 52 integrated with the side port 42, and a proximal end 78 of the helical groove 74 may be proximate the extension tube 52.

In some embodiments, the helical groove 74 may facilitate flushability by inducing more turbulent fluid flow that increases fluid exchange. In some embodiments, the helical groove 74 may terminate just distal of the septum 48, creating a fluid path to the region difficult to flush. In some embodiments, the side port 42 may be molded with a threaded core pin. In some embodiments, in response to the threaded core pin being screwed or unscrewed during molding, the helical groove 74 may be created.

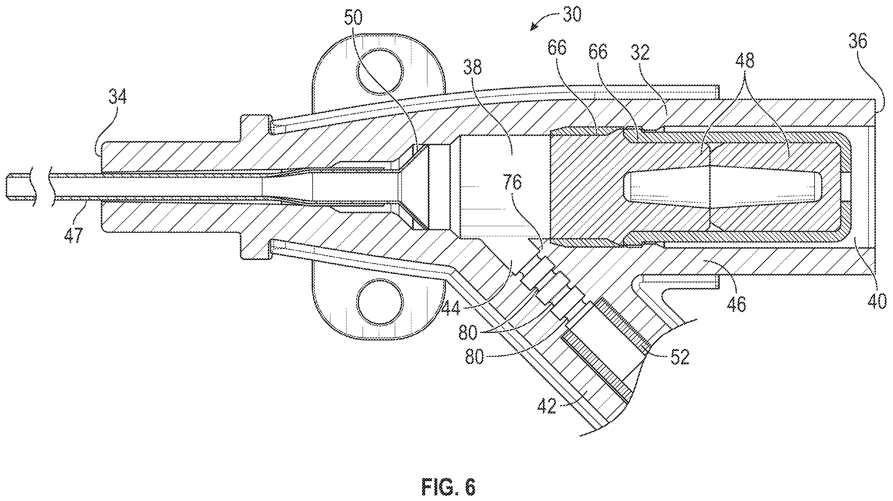

Referring now to Figure 6, in some embodiments, the non-cylindrical portion may include multiple ridges 80, which may be generally perpendicular to a central axis of the side port pathway 44. In some embodiments, the ridges 80 may be evenly spaced apart. In some embodiments, the ridges 80 may be annular. In some embodiments, the ridges 80 may increase pressure and restrict flow, resulting in pulsatile or more turbulent fluid flow.

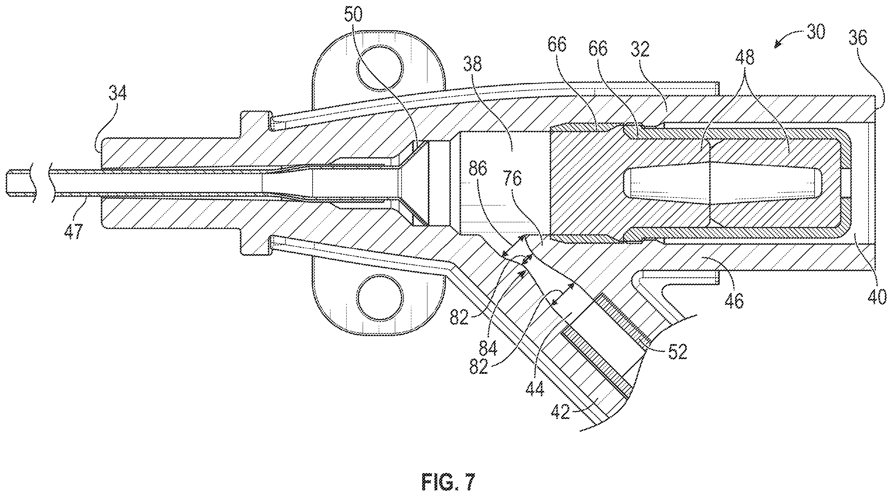

Referring now to Figure 7, in some embodiments, the non-cylindrical portion may include a narrowed diameter portion 82. In some embodiments, the narrowed diameter portion 82 may function as an inlet nozzle. In some embodiments, distal to the narrowed diameter portion 82, the side port pathway 44 may converge in a proximal direction towards the narrowed diameter portion 82, and proximal to the narrowed diameter portion 82, the side port pathway 44 may diverge in the proximal direction away from the narrowed diameter portion 82. In some embodiments, the narrowed diameter portion 82 may include a first diameter 84. In some embodiments, the side port pathway 44 may include a second diameter 86 distal to the first diameter 84 and a third diameter 88 proximal to the first diameter 84. In some embodiments, the second diameter 86 and the third diameter 88 may be greater than the first diameter 84. In some embodiments, the narrowed diameter portion 82 may be annular.

In some embodiments, the narrowed diameter portion 82 may be proximate the lumen 38. In some embodiments, the narrowed diameter portion 82 may be asymmetric. For example, the narrowed diameter portion 82 may include a protrusion only on a distal side of the side port pathway 44. In these and other embodiments, the narrowed diameter portion 82 may facilitate turbulence and a rotational flow and deflection toward the septum 48.

In some embodiments, the non-cylindrical portion may be disposed within the extension tube 52, which may be disposed within the side port pathway 44 and/or may extend to the lumen 40. Thus, in some embodiments, the narrowed diameter portion 82 may be disposed within the extension tube 52. In some embodiments, the extension tube 52 may not extend into the lumen 40.

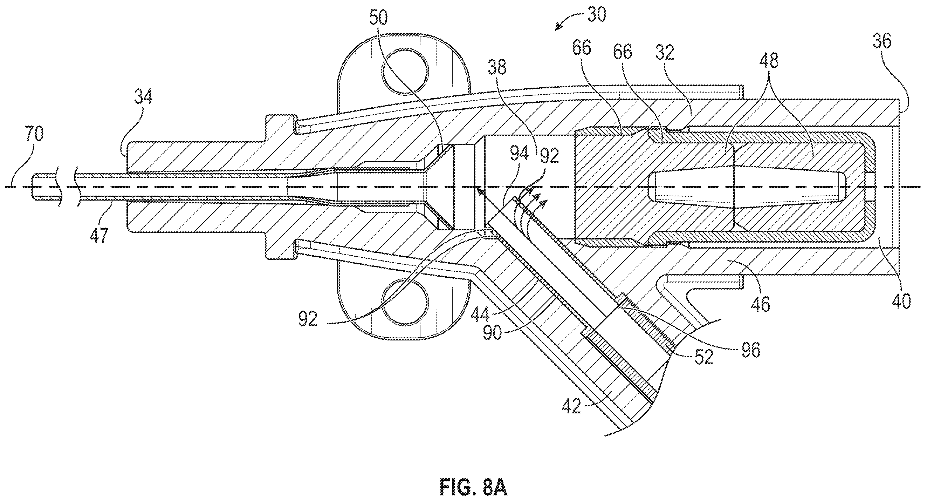

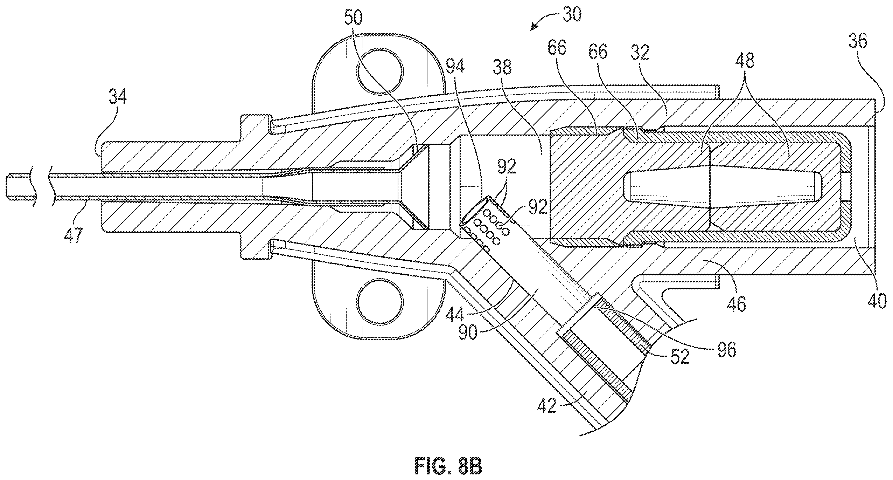

Referring now to Figures 8A-8B, in some embodiments, the catheter assembly 30 may include an insert 90 secured within the side port pathway 44 and configured to divert fluid flow. In some embodiments, the insert 90 may include a fluid pathway therethrough and in fluid communication with the side port pathway 44 and the lumen 40. In some embodiments, the insert 90 may extend into the lumen 40 from the side port pathway 44 and may include one or more perforations or holes 92. In some embodiments, the holes 92 may be configured to divert fluid flow towards the septum 48 and into the region difficult to flush.

In some embodiments, a distal end of the insert 90 disposed within the lumen 40 may include the holes 92. In some embodiments, a proximal end 96 of the insert 90 may be adjacent and/or contacting the extension tube 52. In some embodiments, the insert 90 may be tubular or conical. In some embodiments, the insert 90 may include an opening 94 aligned with the central axis of the side port pathway 44 and/or larger than the holes 92 such that a significant portion of the fluid continues in the distal direction. In some embodiments, the term "insert" may refer to an element that is inserted within the catheter adapter 32 and is not monolithically formed with the catheter adapter as a single unit. In some embodiments, the insert 90 may be formed by a distal end of the extension tube 52, which may extend into or through the side port pathway 44 and which may divert fluid flow.

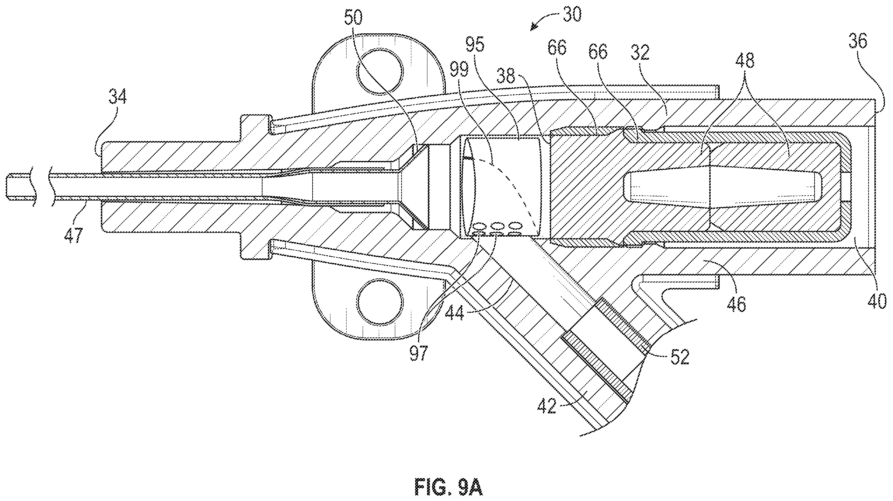

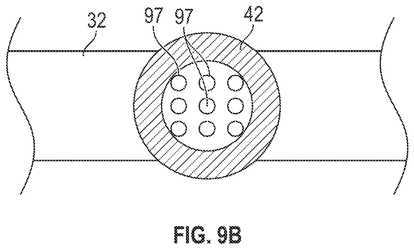

Referring now to Figures 9A-9B, in some embodiments, an insert 95 may include a ring, which may include one or more holes 97 aligned with the side port pathway 44. In some embodiments, the holes 97 may be configured to divert fluid flow, which may flow towards the septum 48. In some embodiments, the ring may include a channel therethrough such that an introducer needle (not illustrated) may extend through the ring and the catheter 47. In some embodiments, the channel may be aligned with the longitudinal axis 70 (see, for example, Figure 4A) of the catheter assembly 30. In some embodiments, an inner surface of the ring may include a groove or a ridge 99, which may guide an instrument inserted through the side port 42, a particular one of the holes 97, and into and/or through the catheter 47. In some embodiments, the instrument may include a probe, obturator, introducer, catheter, or another suitable instrument.

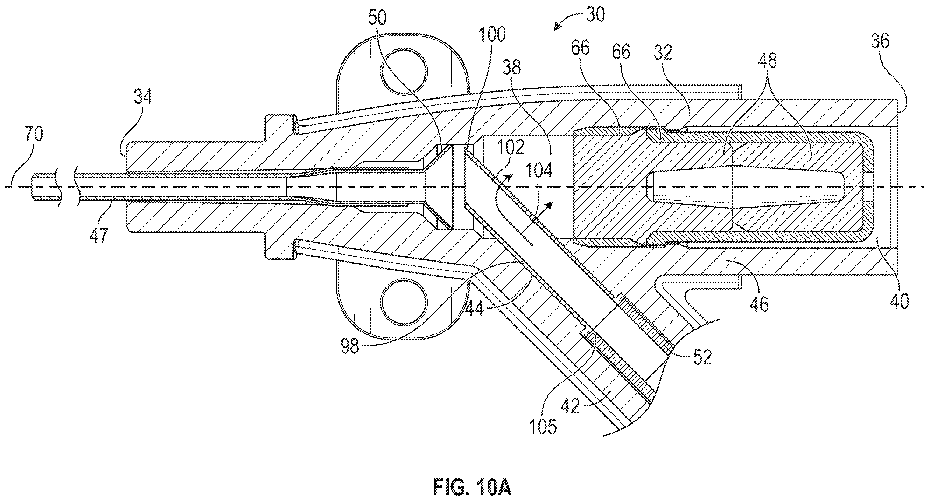

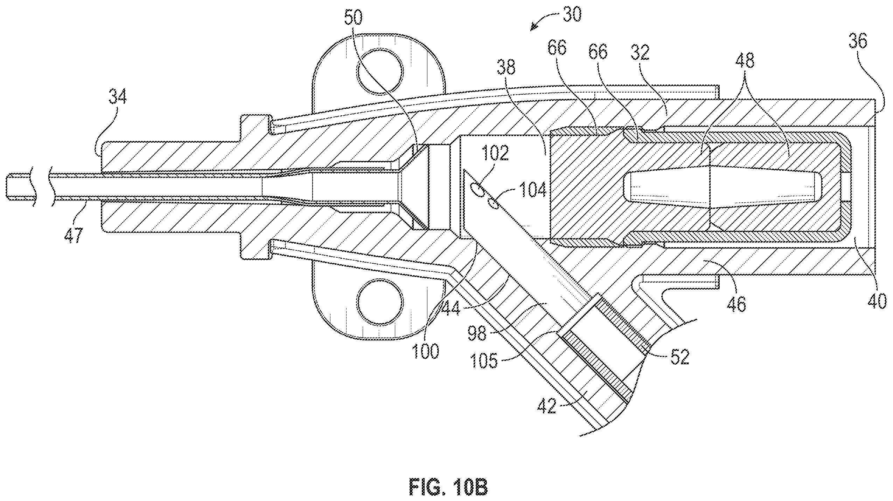

Referring now to Figures 10A-10B, in some embodiments, the catheter assembly 30 may include an insert 98 secured within the side port pathway 44 and configured to divert fluid flow. In some embodiments, the insert 98 may include a fluid pathway therethrough and in fluid communication with the side port pathway 44 and the lumen 40. In some embodiments, the insert 98 may extend into the lumen 40 from the side port pathway 44. In some embodiments, the insert 98 may include a cannula, which may include a distal end generally perpendicular to the longitudinal axis 70 (see, for example, Figure 4A) of the catheter assembly 30. In some embodiments, the distal end of the insert 98 may include an opening 100 and may be configured to direct fluid distally.

In some embodiments, the cannula may include a first side hole 102 and a second side hole 104. In some embodiments, the first side hole 102 may be aligned with the opening 100, and the first side hole 102 and the opening 100 may be configured to receive the introducer needle therethrough. In some embodiments, the second side hole 104 may be configured to direct fluid from the side port 42 proximally toward the septum 48 and the region difficult to flush. In some embodiments, the second side hole 104 may be proximal to the first side hole 102. In some embodiments, a proximal end 105 of the insert 98 may be adjacent and/or contacting the extension tube 52.

In some embodiments, the insert 98 may be formed by a distal end of the extension tube 52, which may extend into or through the side port pathway 44 and which may divert fluid flow. Thus, in some embodiments, the insert 98 and the extension tube 52 may be monolithically formed as a single unit.

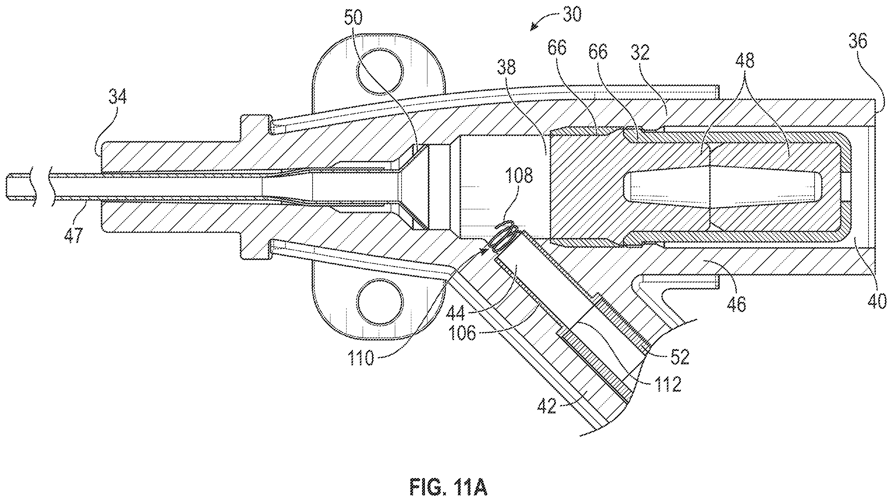

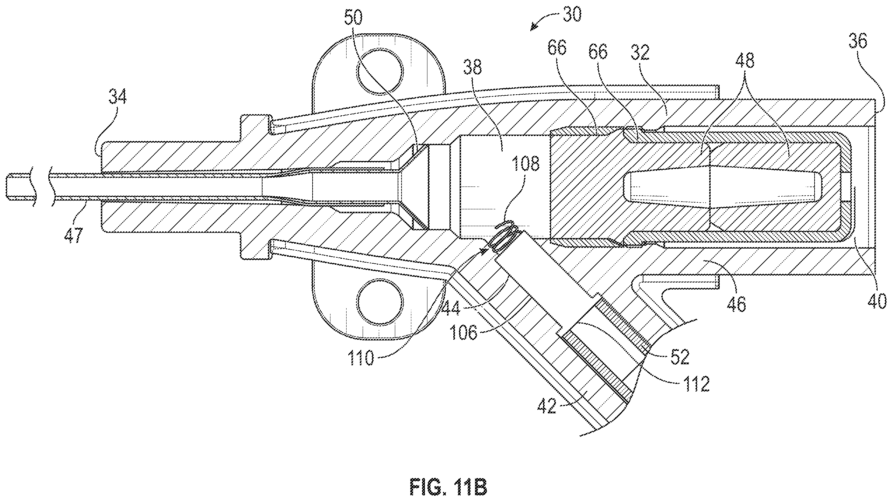

Referring now to Figures 11A-11B, in some embodiments, the catheter assembly 30 may include an insert 106 secured within the side port pathway 44. In some embodiments, a distal end 110 of the insert 106 may include a corkscrew shape 108, which may be helical. In some embodiments, the corkscrew shape 108 may include multiple coils, which may be disposed within the lumen 40 and provide a guide for the instrument inserted through the side port 42. In some embodiments, the corkscrew shape 108 may divert fluid flow from the side port pathway 44 and may facilitate flushing of the region. In some embodiments, the insert 106 may include a fluid pathway therethrough and in fluid communication with the side port pathway 44 and the lumen 40. In some embodiments, the insert 106 may extend into the lumen 40 from the side port pathway 44. In some embodiments, the insert 106 may include a cannula, and the corkscrew shape 108 may extend from a distal end of the cannula. In some embodiments, the distal end of the cannula may include an opening and may be configured to direct fluid distally. In some embodiments, a proximal end 112 of the insert 106 may be adjacent and/or contacting the extension tube 52.

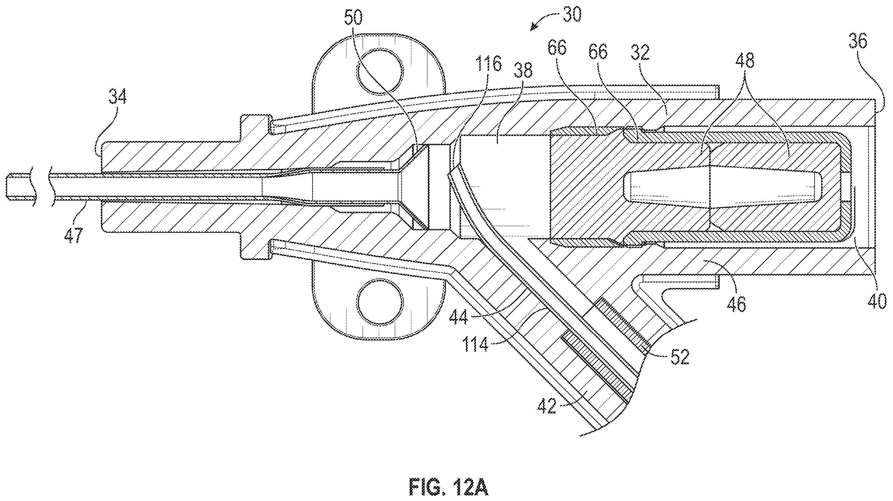

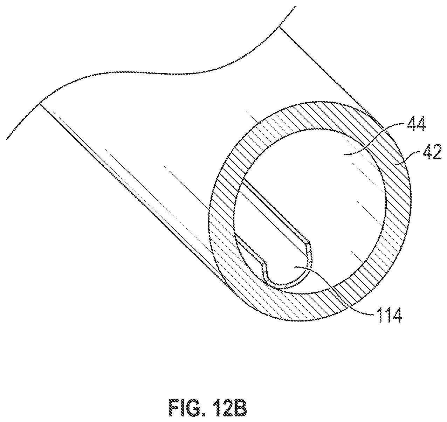

Referring now to Figures 12A-12B, in some embodiments, the catheter assembly 30 may include an insert 114, which may include a groove extending along a longitudinal axis of the insert 114. In some embodiments, the groove may be semi-circular.

In some embodiments, the insert 114 may be advanced through the side port 42 and into the catheter 47 immediately prior to flushing. In some embodiments, during flushing, the groove may direct fluid flow toward the septum 48 and the region otherwise difficult to flush. In some embodiments, after completion of flushing, the insert 114 may be removed from the catheter assembly 30 so as to not interfere with infusion or aspiration.

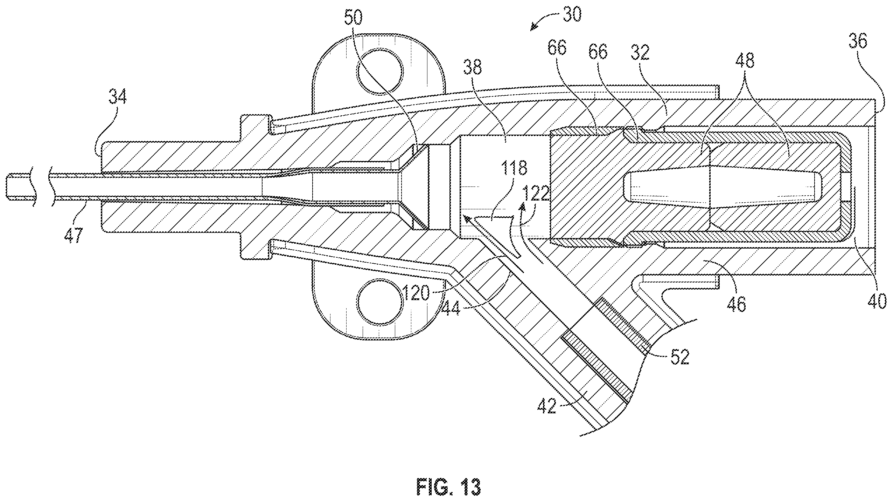

Referring now to Figure 13, in some embodiments, the catheter assembly may include a insert 118 that may be coupled to the inner surface 38. In some embodiments, the insert 118 may include a protrusion, which may include a generally triangular shape. In some embodiments, the protrusion may be monolithically formed with the inner surface 38 as a single unit instead of being inserted into the lumen 40. In some embodiments, the protrusion may divide the side port pathway 44 into a first fluid pathway 120 extending in a distal direction and a second fluid pathway 122 extending in a proximal direction towards the septum 48.

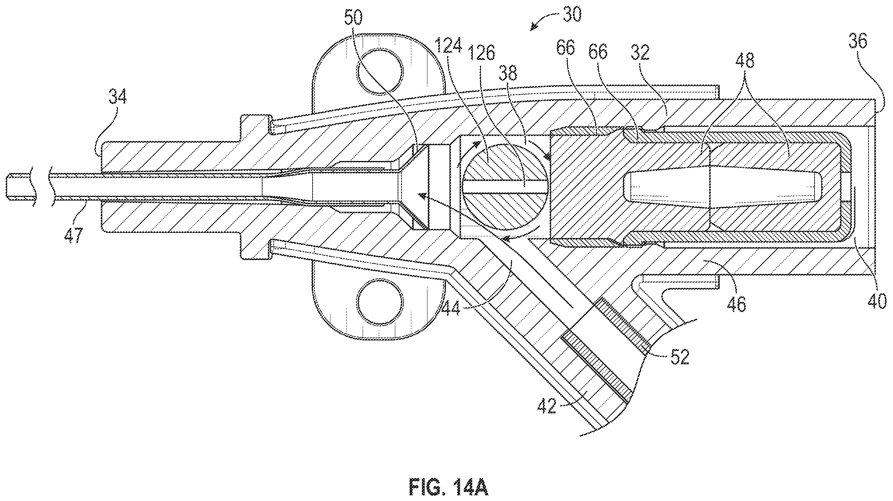

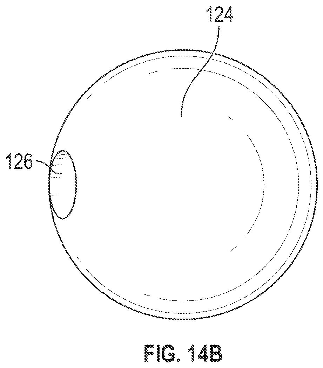

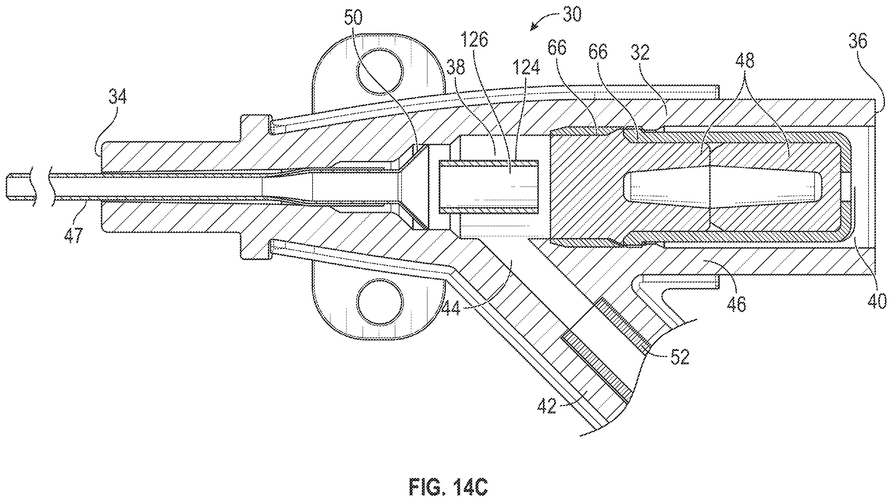

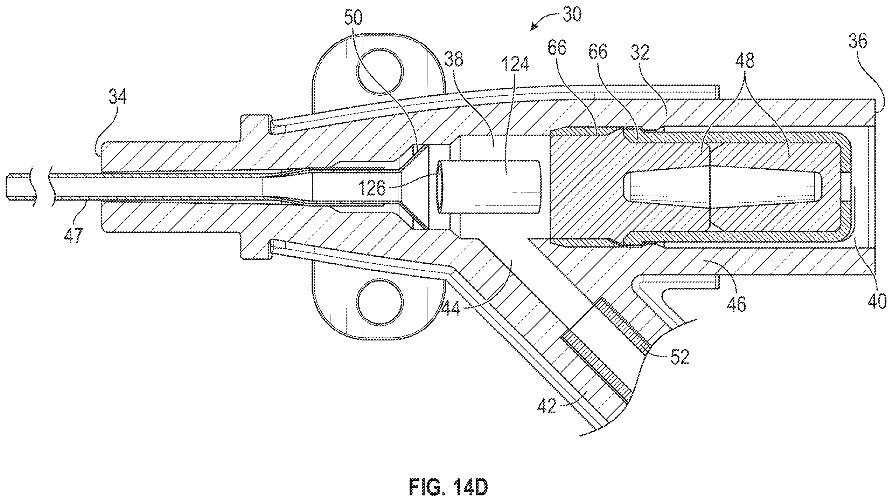

Referring now to Figures 14A-14D, in some embodiments, the catheter assembly 30 may include a fluid diverter 124 disposed between the septum 48 and the catheter 47 and aligned with the side port pathway 44. In some embodiments, the fluid diverter 124 may include a rotation element, which may include a straight pathway 126 extending therethrough. In some embodiments, the introducer needle may extend through the straight pathway 126, which may limit rotation of the rotation element. In some embodiments, the rotation element may be a cylinder, as illustrated, for example, in Figures 14C-14D, or a sphere, as illustrated, for example in Figures 14A-14B. In some embodiments, in response to the introducer needle being removed from the catheter assembly 30, the rotation element may be configured to freely rotate or spin in response to infusion of fluid from the side port pathway 44 to the lumen 40, redirecting fluid and improving flushability. In some embodiments, the rotation element may be configured to float in the fluid in the lumen 40. In some embodiments, the rotation element may not be coupled to the catheter adapter 32 and may be unfixed. In some embodiments, the rotation element may be coated with an anti-microbial coating to prevent bacterial growth within the catheter adapter 32.

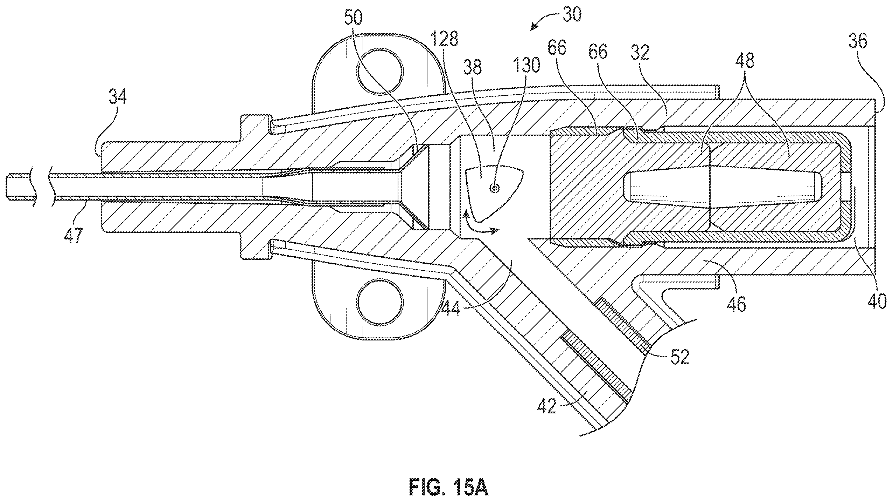

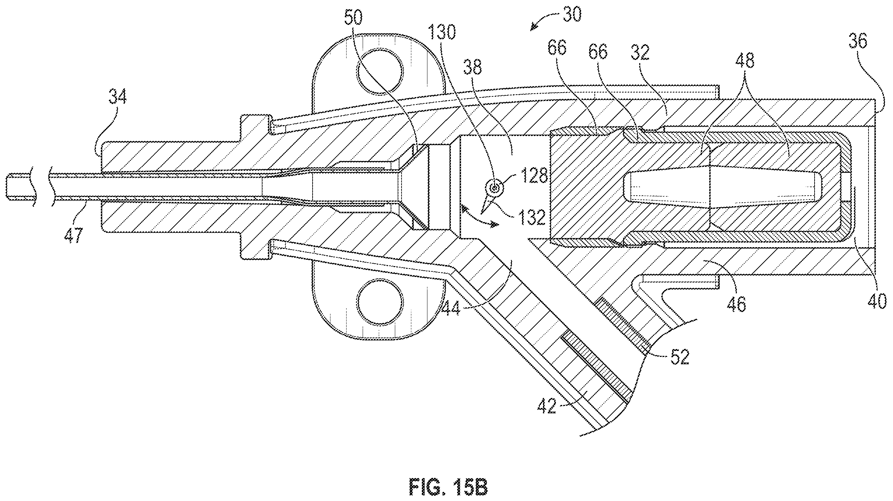

Referring now to Figures 15A-16B, in some embodiments, a fluid diverter may include a pivot element 128 coupled to the inner surface 38 and configured to pivot. In some embodiments, the pivot element 128 may pivot about a pin 130, which may couple the pivot element 128 to the inner surface 38. In some embodiments, the pivot element 128 may be generally triangular shaped, as illustrated, for example, in Figures 15A-15B. As illustrated in Figures 16A-16B, the pivot element 128 may include an elongated tab 132 extending outwardly from a body of the pivot element 128. In some embodiments, the pivot element 128 may include a door or opening to allow a separate device, such as the instrument, to be passed through. In some embodiments, the pivot element 128 may be configured to pivot in response to insertion of the instrument or other device distally through the side port 42.

All examples and conditional language recited herein are intended for pedagogical objects to aid the reader in understanding the invention and the concepts contributed by the inventor to furthering the art and are to be construed as being without limitation to such specifically recited examples and conditions. Although embodiments of the present inventions have been described in detail, it should be understood that the various changes, substitutions, and alterations could be made hereto without departing from the spirit and scope of the invention.

### FURTHER ASPECTS

**[Aspect** 1] A catheter assembly, comprising: a catheter adapter, comprising a distal end, a proximal end, an inner surface extending through the distal end and the proximal end and forming a lumen, and a side port forming a side port pathway through a sidewall of the catheter adapter and in fluid communication with the lumen; an extension tube integrated within the side port, wherein a diameter of the side port pathway increases in a distal direction; a catheter extending distally from the distal end of the catheter adapter; and a septum disposed within the lumen proximal to the side port pathway.

**[Aspect** 2] The catheter assembly of aspect 1, wherein the side port pathway comprises a first side and a second side opposite the first side, wherein the first side is straight, wherein a proximal end of the second side is straight and a distal end of the second side is angled proximally with respect to the proximal end of the second side, wherein the first side is distal to the second side.

**[Aspect 3]** The catheter assembly of aspect 2, wherein the distal end of the second side contacts the septum.

**[Aspect 4]** The catheter assembly of aspect 3, wherein the septum comprises an elastomeric body surrounded by a canister.

**[Aspect 5]** A catheter assembly, comprising: a catheter adapter, comprising a distal end, a proximal end, an inner surface extending through the distal end and the proximal end and forming a lumen, and a side port forming a side port pathway through a sidewall of the catheter adapter and in fluid communication with the lumen; a catheter extending distally from the distal end of the catheter adapter; and a septum disposed within the lumen proximal to the side port pathway, wherein the side port pathway comprises a non-cylindrical portion.

[Aspect 6] The catheter assembly of aspect 5, wherein the non-cylindrical portion comprises a helical groove.

[Aspect 7] The catheter assembly of aspect 6, wherein a distal end of the helical groove is proximate the septum.

[Aspect 8] The catheter assembly of aspect 7, wherein the septum comprises an elastomeric body surrounded by a canister.

**[Aspect 9]** The catheter assembly of aspect 6, wherein the catheter assembly comprises an extension tube integrated with the side port, wherein a proximal end of the helical groove is proximate the extension tube.

[Aspect 10] The catheter assembly of aspect 5, wherein the non-cylindrical portion comprises a plurality of ridges generally perpendicular to a central axis of the side port pathway.

**[Aspect** 11] The catheter assembly of aspect 10, wherein the plurality of ridges are evenly spaced apart.

**[Aspect 12]** The catheter assembly of aspect 10, wherein each of the plurality of ridges are annular.

**[Aspect** 13] The catheter assembly of aspect 5, wherein the non-cylindrical portion comprises a narrowed diameter portion.

**[Aspect 14]** The catheter assembly of aspect 13, wherein the narrowed diameter portion comprises a first diameter, wherein the side port pathway comprises a second diameter distal to the first diameter and a third diameter proximal to the first diameter, wherein the second diameter and the third diameter are greater than the first diameter.

**[Aspect 15]** The catheter assembly of aspect 5, wherein the non-cylindrical portion comprises a plurality of ridges or grooves extending generally parallel to a central axis of the side port pathway.

**[Aspect 16]** The catheter assembly of aspect 15, wherein the plurality of grooves are evenly spaced around an inner circumference of the side port.

**[Aspect 17]** The catheter assembly of aspect 5, wherein the catheter assembly comprises an extension tube integrated with the side port, wherein each of the plurality of grooves comprises a proximal end proximate the extension tube.

[Aspect 18] The catheter assembly of aspect 7, wherein each of the plurality of grooves comprises a distal end proximate the lumen.

[Aspect 19] A catheter assembly, comprising: a catheter adapter, comprising a distal end, a proximal end, an inner surface extending through the distal end and the proximal end and forming a lumen, and a side port forming a side port pathway through a sidewall of the catheter adapter and in fluid communication with the lumen; a catheter extending distally from the distal end of the catheter adapter; and a septum disposed within the lumen proximal to the side port pathway; an insert disposed at least partially within the side port pathway and configured to divert fluid flow.

[Aspect 20] The catheter assembly of aspect 19, wherein the insert extends into the lumen from the side port pathway and comprises a plurality of holes.

[Aspect 21] The catheter assembly of aspect 20, wherein the insert is formed by a distal end of an extension tube extending through the side port pathway and into the lumen.

**[Aspect 22]** The catheter assembly of aspect 19, wherein a distal end of the insert comprises a corkscrew shape.

**[Aspect 23]** The catheter assembly of aspect 19, wherein the insert comprises a groove extending along a longitudinal axis of the insert.

[Aspect 24] The catheter assembly of aspect 19, wherein the insert comprises a protrusion on the inner surface, wherein the protrusion divides the side port pathway into a first fluid pathway extending in a distal direction and a second fluid pathway extending in a proximal direction.

**[Aspect 25]** A catheter assembly, comprising: a catheter adapter, comprising a distal end, a proximal end, an inner surface extending through the distal end and the proximal end and forming a lumen, and a side port forming a side port pathway through a sidewall of the catheter adapter and in fluid communication with the lumen; a catheter extending distally from the distal end of the catheter adapter; and a septum disposed within the lumen proximal to the side port pathway; a fluid diverter disposed between the septum and the catheter and aligned with the side port pathway, the fluid diverter comprising: a rotation element comprising a straight pathway extending therethrough and further comprising a cylinder or a sphere, wherein the rotation element is configured to rotate in response to infusion of fluid from the side port pathway to the lumen; or a pivot element coupled to the inner surface and configured to pivot.

## Claims

1. A catheter assembly, comprising:
a catheter adapter, comprising a distal end, a proximal end, an inner surface extending through the distal end and the proximal end and forming a lumen, and a side port forming a side port pathway through a sidewall of the catheter adapter and in fluid communication with the lumen;
a catheter extending distally from the distal end of the catheter adapter; and
a septum disposed within the lumen proximal to the side port pathway,
**characterized in that** the side port pathway comprises a non-cylindrical portion.

2. The catheter assembly of claim 1, wherein the non-cylindrical portion comprises a helical groove.

3. The catheter assembly of claim 2, wherein a distal end of the helical groove is proximate the septum.

4. The catheter assembly of claim 3, wherein the septum comprises an elastomeric body surrounded by a canister.

5. The catheter assembly of claim 2, wherein the catheter assembly comprises an extension tube integrated with the side port, wherein a proximal end of the helical groove is proximate the extension tube.

6. The catheter assembly of claim 1, wherein the non-cylindrical portion comprises a plurality of ridges generally perpendicular to a central axis of the side port pathway.

7. The catheter assembly of claim 6, wherein the plurality of ridges are evenly spaced apart.

8. The catheter assembly of claim 6, wherein each of the plurality of ridges are annular.

9. The catheter assembly of claim 1, wherein the non-cylindrical portion comprises a narrowed diameter portion.

10. The catheter assembly of claim 9, wherein the narrowed diameter portion comprises a first diameter, wherein the side port pathway comprises a second diameter distal to the first diameter and a third diameter proximal to the first diameter, wherein the second diameter and the third diameter are greater than the first diameter.

11. The catheter assembly of claim 1, wherein the non-cylindrical portion comprises a plurality of ridges or grooves extending generally parallel to a central axis of the side port pathway.

12. The catheter assembly of claim 11, wherein the plurality of ridge or grooves are evenly spaced around an inner circumference of the side port.

13. The catheter assembly of claim 1, wherein the catheter assembly comprises an extension tube integrated with the side port, and wherein the non-cylindrical portion comprises a plurality of grooves, with each of the plurality of grooves comprising a proximal end proximate the extension tube.

14. The catheter assembly of claim 1, wherein the non-cylindrical portion comprises a plurality of grooves, and wherein each of the plurality of grooves comprises a distal end proximate the lumen.

15. A catheter assembly, comprising:
a catheter adapter, comprising a distal end, a proximal end, an inner surface extending through the distal end and the proximal end and forming a lumen, and a side port forming a side port pathway through a sidewall of the catheter adapter and in fluid communication with the lumen;
a catheter extending distally from the distal end of the catheter adapter; and
a septum disposed within the lumen proximal to the side port pathway;
**characterized in that** the catheter assembly further includes an insert disposed at least partially within the side port pathway and configured to divert fluid flow.

16. The catheter assembly of claim 15, wherein the insert extends into the lumen from the side port pathway and comprises a plurality of holes.

17. The catheter assembly of claim 16, wherein the insert is formed by a distal end of an extension tube extending through the side port pathway and into the lumen.

18. The catheter assembly of claim 15, wherein a distal end of the insert comprises a corkscrew shape.

19. The catheter assembly of claim 15, wherein the insert comprises a groove extending along a longitudinal axis of the insert.

20. The catheter assembly of claim 15, wherein the insert comprises a protrusion on the inner surface, wherein the protrusion divides the side port pathway into a first fluid pathway extending in a distal direction and a second fluid pathway extending in a proximal direction.

21. A catheter assembly, comprising:
a catheter adapter, comprising a distal end, a proximal end, an inner surface extending through the distal end and the proximal end and forming a lumen, and a side port forming a side port pathway through a sidewall of the catheter adapter and in fluid communication with the lumen;
a catheter extending distally from the distal end of the catheter adapter; and
a septum disposed within the lumen proximal to the side port pathway;
a fluid diverter disposed between the septum and the catheter and aligned with the side port pathway,
**characterized in that** the fluid diverter comprises:
a rotation element comprising a straight pathway extending therethrough and further comprising a cylinder or a sphere, wherein the rotation element is configured to rotate in response to infusion of fluid from the side port pathway to the lumen; or
a pivot element coupled to the inner surface and configured to pivot.
